# EUROPÄISCHE PATENTSCHRIFT

(11) **EP 2 895 858 B1**
(45) Veröffentlichungstag und Bekanntmachung des Hinweises auf die Patenterteilung: **23.11.2016**
(21) Anmeldenummer: 13760057.3
(22) Anmeldetag: 13.09.2013
(51) Int. Cl.: C12Q 1/26, A61K 47/48, G01N 33/532

(54) **VERFAHREN ZUM MARKIEREN ODER IMMOBILISIEREN EINER ZIELSTRUKTUR**
METHOD FOR MARKING OR IMMOBILIZING A TARGET STRUCTURE
PROCÉDÉ DE MARQUAGE OU IMMOBILISATION D'UNE STRUCTURE CIBLE

(30) Priorität: 13.09.2012 DE 102012216346; 14.09.2012 US 201261701133 P
(43) Veröffentlichungstag der Anmeldung: 22.07.2015
(73) Patentinhaber: Fraunhofer-Gesellschaft zur Förderung der angewandten Forschung e.V., 80686 München (DE)
(72) Erfinder: SZARDENINGS, Michael, 04299 Leipzig (DE); GRUNWALD, Ingo, 28865 Lilienthal (DE); RISCHKA, Klaus, 21255 Tostedt (DE); RICHTER, Katharina, 28215 Bremen (DE)
(74) Vertreter: Eisenführ Speiser
(86) Internationale Anmeldenummer: PCT/EP2013/069016
(87) Internationale Veröffentlichungsnummer: WO 2014/041126

(56) Entgegenhaltungen:
- WO-A2-2011/153250
- NICKOLOFF B J ET AL: "AFFINITY-DEPENDENT CROSS-LINKING TO NEUROTOXIN SITES OF THE ACETYLCHOLINE RECEPTOR MEDIATED BY CATECHOL OXIDATION", BIOCHEMISTRY, AMERICAN CHEMICAL SOCIETY, US, Bd. 24, Nr. 4, 1. Januar 1985 (1985-01-01) , Seiten 999-1007, XP002062134, ISSN: 0006-2960, DOI: 10.1021/BI00325A029
- GALLOP P M ET AL: "IS THE ANTIOXIDANT, ANTI-INFLAMMATORY PUTATIVE NEW VITAMIN, PQQ, INVOLVED WITH NITRIC OXIDE IN BONE METABOLISM?", CONNECTIVE TISSUE RESEARCH : AN INTERNAT. JOURNAL, NEW YORK, NY : INFORMA HEALTHCARE, US, Bd. 29, Nr. 2, 1. Januar 1993 (1993-01-01) , Seiten 153-161, XP002068028, ISSN: 0300-8207, DOI: 10.3109/03008209309014242
- Bioresources Com ET AL: "PEER-REVIEWED REVIEW ARTICLE LACCASE: PROPERTIES AND APPLICATIONS", BioResources, 1. Januar 2009 (2009-01-01), Seiten 1694-1717, XP055087912, Gefunden im Internet: URL:http://www.ncsu.edu/bioresources/BioRe s_04/BioRes_04_4_1694_Madhavi_Lele_Laccase _Properties_Applications_Review_567.pdf [gefunden am 2013-11-12]

## Beschreibung

Die Erfindung betrifft ein Verfahren zum Markieren einer Zielstruktur, wobei eine Dihydroxy- oder Trihydroxyphenylgruppe umfassende Verbindung V mittels der Oxidation dieser Gruppe zum Chinon kovalent an eine Zielstruktur gebunden wird. Dabei sind die Konzentrationsbedingungen der Dihydroxy- oder Trihydroxyphenylgruppe enthaltenden Verbindung so eingestellt, dass nur ein ganz geringer Teil der Chinongruppen so miteinander reagiert, dass eine Dimerisierung bzw. Polymerisierung eintritt.

Die Erfindung betrifft auch die Verwendung einer Verbindung V zum Markieren einer Zielstruktur, wobei ein Oxidieren der Dihydroxy- oder Trihydroxyphenylgruppe (bevorzugt durch ein Enzym) erfolgt.

Bei einer Vielzahl von im Stand der Technik bekannten Markierungsmethoden für biologische Strukturen, insbesondere für Zellen, erfolgt die Markierung beispielsweise durch Sonden häufig ohne dass eine kovalente Bindung zwischen markierendem und zu markierendem Konstrukt entsteht. Damit die Bindung zwischen markierendem und zu markierendem Konstrukt hinreichend lange stabil bleibt, so dass zum Beispiel Messungen durchgeführt oder zum Beispiel biochemische oder biologische Reaktionen stattfinden, sind in aller Regel Affinitäten mit Dissoziationskonstanten im subnanomolaren Bereich erforderlich, die Halbwertszeiten des gebildeten Komplexes im Bereich von zumindest mehreren Minuten aufweisen. In aller Regel verfügen nur Antikörper über eine derartige Affinität und deshalb sind sie die bevorzugten Reagenzien für Verfahren wie zum Beispiel die Markierung oder Sortierung von Zellen. Viele andere mögliche Moleküle zur Markierung, wie zum Beispiel Peptide, weisen zwar eine ähnlich hohe Spezifität für den zu markierenden Bindungspartner auf, die Bindungskonstanten und damit die Halbwertszeit eines gebildeten Komplexes sind jedoch nicht ausreichend für die meisten Verfahren. Dies bedeutet, dass das Bindungsgleichgewicht verglichen mit kovalenten Bindungen stärker in Richtung der ungebundenen Partner verschoben ist. Daraus folgt, dass höhere Konzentrationen an markierender Struktur eingesetzt werden müssen, um ein gewünschtes Markierungsergebnis zu erzielen. Dies führt auch zu einem erhöhten Hintergrundsignal. Bei Erzeugung einer (irreversiblen) kovalenten Bindung zwischen den Bindungspartnern spielt die Stabilität des Komplexes im Prinzip keine Rolle mehr für den Erfolg der Durchführung von Markierungsexperimenten, da es möglich ist, den ungebunden verbliebenen Teil der Markierungsmoleküle abzutrennen, ohne die Signalstärke der Markierungsreaktion zu beeinträchtigen.

Eine schonende kovalente Markierung von Molekülen, insbesondere von Zelloberflächenmarkern in vitro und in vivo, ist nach heutigem Stand der Technik nur mit chemisch aktivierten und zumeist instabilen Markierungssonden möglich.

Hierzu ist es im Allgemeinen üblich, reaktive funktionelle Gruppen zu erzeugen. Dazu müssen aber z.B. bei der Aktivierung von Carbonsäuren mit EDC/NHS mehrere Schritte durchlaufen werden. Insbesondere muss die aktivierte Carbonsäure häufig von den Reagenzien gereinigt werden, ein Vorgang bei dem die instabile aktivierte Gruppe auch wieder zerfällt (Review: Hackenberger CP, Schwarzer D. (2008): "Chemoselective ligation and modification strategies for peptides and proteins". Angew Chem Int Ed Engl. 2008; 47(52): 10030-74).

Eine andere Alternative stellt die sogenannte Click-Chemie dar, bei der neuerdings auch unter physiologischen Bedingungen zwei Moleküle miteinander kovalent gekoppelt werden können. Diese Methode setzt allerdings voraus, dass beide zu koppelnden Moleküle vorab modifiziert werden. Die Modifikation beider Moleküle ist aber anders als in der hier beschriebenen Erfindung wesentlich aufwendiger, in vielen Fällen sogar unmöglich. Der Vorteil dieser Methode, der auch das sehr positive Echo in Fachkreisen erklärt, ist aber die Stabilität der Ausgangsverbindungen und die sehr schnelle und höchst spezifische Reaktion, die es wie im Fall der hier beschriebenen Erfindung zulässt, dass in die Reaktion nur zumindest ein genau definierbarer Reaktionspartner eingeht. Die Reaktion der Partner setzt in vielen Fällen auch nicht die hier ausgeführte Struktur eines bindenden Moleküls voraus und ist daher zum Beispiel zur Bestimmung von Konzentrationen unbekannter Mengen des zu markierenden Objekts ungeeignet. (Review: Sletten EM, Bertozzi CR.: "Bioorthogonal chemistry: fishing for selectivity in a sea of functionality." Angew Chem Int Ed Engl. 2009; 48(3(:6974-98).

Aus dem Stand der Technik ist ferner bekannt, dass zum Beispiel die Muschel *Mytilus edulis* in der Lage ist, sich mit Hilfe von proteinogenen Byssus-Fäden und Klebstoffplaques an eine Vielzahl von Oberflächen anzuhaften. Zu diesen Oberflächen zählen Glas, Metall, Holz, Kunststoff und sogar Teflon. Der Klebstoff besteht aus unterschiedlichen Proteinen, die als Mefp (*Mytilus edulis* food proteine) bezeichnet werden. Mefp-1 ist essenzieller Bestandteil der äußeren Beschichtung des Byssus und der Plaques. Bemerkenswert ist die Zusammensetzung dieses Proteins. Es handelt sich dabei um ein repetitives Dekapeptid mit einer molekularen Masse von 108 kDa. Für den eigentlichen Klebvorgang sind Mefp-3 und Mefp-5 verantwortlich. Diese Proteine sind in der Grenzschicht zu den Substratoberflächen lokalisiert. Mefp-2 ist verantwortlich für die schaumartige Morphologie und die Stabilisierung des Klebstoffes. Die Kollagenfasern des Byssus werden durch Mefp-4 mit den Plaqueproteinen verbunden. Besonders bemerkenswert ist der DOPA-Anteil im Klebstoff (10 bis 27 Mol-%).

DOPA ist durch seine mannigfaltigen Reaktionsmöglichkeiten eine Schlüsselaminosäure des Miesmuschelklebstoffes sowohl für die Adhäsion als auch für die Kohäsion. Entscheidend für die Kohäsion sind kovalente Vernetzungen, die zum einen durch die sog. 1,4-Michael-Addition mit Aminogruppen in der Lysin-Seitenketten sowie durch die Bindung einer Schiff'schen Base (Imin) und zum anderen durch die radikalische Dimerisierung der Catecholreste über die Zwischenstufe eines Chinons untereinander etabliert werden. Die Catecholuntereinheit von DOPA ist ferner in der Lage, äußerst stabile Metallkomplexe auszubilden, die ebenfalls zur Stabilisierung des Klebstoffes entscheidend beitragen. Die Quervernetzungsreaktionen können enzymatisch durch die Zugabe z.B. einer Tyrosinase oder Lakkase sowie durch Oxidationsreagenzien wie NalO₄ oder H₂O₂ initiiert werden.

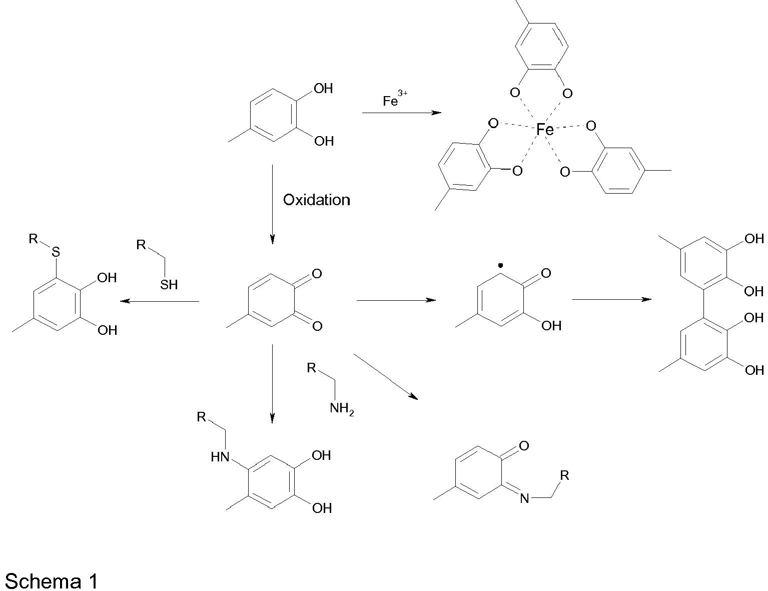

Problematisch bei diesen aus der Natur bekannten Anhaftungsphänomenen ist, dass eine Vielzahl von Nebenreaktionen und Polymerisierungsreaktionen nicht nur möglich sind, sondern auch tatsächlich stattfinden (vgl. Schema 1). Damit galt das System bislang als nicht ausreichend beherrschbar für die gezielte Markierung von Zielstrukturen. Die Verwendung in relevanten meßtechnischen Verfahren, bei denen aus regulatorischen Gründen die Bedingungen der Reaktion und die verwendeten Moleküle genau definiert sein müssen, war daher für die Fachwelt nicht einsetzbar. Dazu gehören zum Beispiel die durch entsprechende ISO-Normen geregelten Verfahren der Medizindiagnostik, Medizintechnik und Transplantationsverfahren.

Vor diesem Hintergrund war es Aufgabe der vorliegenden Erfindung, ein Verfahren anzugeben, mit dem zusätzliche zum Stand der Technik bekannte Markierungsmethoden für insbesondere biologische Zielstrukturen ermöglicht werden. Ferner sollte das Verfahren bevorzugt so mild ausgestaltbar sein, dass sogar intakte Zellen ohne Integritätsverlust markiert werden können.

Erfindungsgemäß wird diese Aufgabe gelöst durch ein Verfahren zum Markieren einer Zielstruktur, umfassend die Schritte:
a) Bereitstellen einer Verbindung V, die wenigstens eine Dihydroxy- oder Trihydroxyphenylgruppe umfasst,
b) Bereitstellen eines Mittels zum Umwandeln der Dihydroxy- oder Trihydroxyphenylgruppe zu einer Chinongruppe,
c) Bereitstellen einer biologischen Zielstruktur,
d) Oxidieren der Dihydroxy- oder Trihydroxyphenylgruppe der Verbindung V zur Chinongruppe und
e) Kontaktieren der Verbindung V mit der biologischen Zielstruktur zum Markieren derselben, so dass eine kovalente Verbindung gebildet werden kann,
dadurch gekennzeichnet,
dass im Schritt a) die Verbindung V eine spezifische Erkennungssequenz für die biologische Zielstruktur umfasst,
dass im Schritt b) das Mittel ein Enzym, vorzugsweise eine Oxidoreduktase, ist, und
dass im Schritt e) die Verbindung V in einer Konzentration eingesetzt wird, so dass die maximale Konzentration an Dihydroxy-, Trihydroxyphenylgruppen und Chinongruppen, die durch die Verbindung V eingebracht wurden, ≤ 500µM, bevorzugt ≤ 300µM, weiter bevorzugt ≤ 100µM ist. Dabei gilt bevorzugt-falls Dihydroxy-, Trihydroxyphenyl und/oder Chinongruppen gleichzeitig vorhanden sind, dass die gennanten Konzentrationen als Summe über die Einzelkonzentrationen der genannten Gruppen gelten.

Unter Markieren im Sinne der vorliegenden Erfindung ist ein gezieltes Anbinden einer Verbindung (hier Verbindung V) an eine Zielstruktur über spezifische Erkennungssequenzen zu verstehen. Spezifische Erkennungssequenzen sind dabei solche, die aufgrund ihrer räumlichen Struktur eine erhöhte Affinität zur Zielstruktur oder zu Strukturen an der Oberfläche der Zielstruktur besitzen. Bevorzugte spezifische Erkennungssequenzen sind in diesem Zusammenhang DNA-Sequenzen, RNA-Sequenzen und Aminosäuresequenzen.

Unter Bereitstellen im Sinne des vorliegenden Textes ist jede Form der Zurverfügungstellung zu verstehen. Insbesondere und insbesondere für viele Fälle auch bevorzugt ist ein Bereitstellen auch eine *in situ*-Erzeugung der bereitzustellenden Verbindung.

Überraschenderweise hat sich herausgestellt, dass mit dem erfindungsgemäßen Verfahren durch eine hinreichende Kontrolle der Konzentration der Verbindung V - sei es als Konzentration in der Lösung oder als Konzentration auf der Oberfläche, auf die immobilisiert werden soll - gute und reproduzierbare Ergebnisse beim Binden an die Zielstruktur erzielt werden können, ohne dass es zu einem unerwünschten Maß an ungesteuerten Nebenreaktionen, insbesondere an Dimerisierungen und Polymerisierungen von zu Chinonen oxidierten Dihydroxy- oder Trihydroxyphenylgruppen kommt.

Bevorzugt ist ein Enzym zum Umwandeln der Dihydroxy- oder Trihydroxyphenylgruppe zu einer Chinongruppe eine Oxidoreduktase (EC 1 nach der Klassifizierung nach dem IntEnz (Integrated relational Enzyme database des Swiss Institute of Bioinformatics (SIB)).

Weiter bevorzugt ist das Enzym zum Umwandeln der Dihydroxy- oder Trihydroxyphenylgruppe zu einer Chinongruppe ausgewählt aus der Gruppe bestehend aus den Enzymklassen nach der oben genannten Einteilung EC 1.10 (Oxidoreduktasen, die an Diphenolen und ähnlichen Substanzen als Donoren agieren) und EC 1.14 (Oxidoreduktasen, die an paarigen Donoren unter Einbau oder Reduktion von molekularem Sauerstoff agieren).

Besonders bevorzugt ist die Oxidoreduktase ausgewählt aus den Gruppen EC 1.10.3 (mit Sauerstoff als Akzeptor) und EC 1.14.18 (mit einer anderen Verbindung als Donor und unter Incorporation eines Sauerstoffatoms) und ganz besonders bevorzugt eine Oxidoreduktase aus den Klassen EC 1.10.3.2 (Lakkasen) und EC 1.14.18.1 (Monophenol Monooxigenase). Am meisten bevorzugt sind Lakkasen.

Es hat sich überraschenderweise herausgestellt, dass die Bindungsgeschwindigkeit des (in der Regel durch Oxidation entstandenen) Chinons sehr gut zu üblichen Bindungsgeschwindigkeiten spezifischer Sequenzen an ihre Zielstrukturen passt: So ist es möglich, in der Praxis auch ein stark reversibles Bindungsereignis an die Zielstruktur quasi "einzufrieren". Dies bedeutet, dass das Gleichgewicht durch die kovalenten Bindungen über das Chinon, die der spezifischen nicht-kovalenten Bindung schnell folgen, deutlich zu Zielstruktur/Bindungsstrukturkomplexen verschoben wird. Hinzu kommt noch, dass die Aktivierung durch Enzyme unter verhältnismäßig milden Bedingungen erfolgen kann (insbesondere bei den bevorzugten Enzymen), so dass auch komplexe Zielstrukturen, wie zum Beispiel Zellen, ohne eines Verlustes ihrer Funktionsfähigkeit schnell, zuverlässig und im Falle des Markierens spezifisch gebunden werden können.

Bei dem erfindungsgemäßen Verfahren ist es insbesondere auch möglich, die aktivierenden Enzyme der Lösung zuzugeben, die auch die potentiellen Zielstrukturen enthält.

Wie bereits oben angedeutet, entsteht die die kovalente Bindung über das Chinon insbesondere über Kopplung an Amino- oder Thiogruppen, die regelmäßig in (biologischen) Zielstrukturen vorhanden sind.

Bevorzugt ist ein erfindungsgemäßes Verfahren, wobei die wenigstens eine Dihydroxy- oder Trihydroxyphenylgruppe Bestandteil von DOPA, Dopamin, Kaffeesäure oder einer entsprechenden gebundenen Gruppe aus einer dieser Verbindungen (das heisst, dass DOPA, Dopamin oder Kaffeesäure als Rest an ein Molekül gebunden sind) ist.

Selbstverständlich versteht der Fachmann, dass es sich bei den bevorzugten, die Dihydroxygruppe tragenden Bestandteilen, auch um entsprechende Reste handeln kann. Ebenso ist selbstverständlich, dass die entsprechenden Dihydroxygruppe tragenden Bestandteile auch *in situ* z.B. durch Oxidation eines Tyrosinrestes erzeugt werden können. Mit den bevorzugten, Catecholgruppen tragenden Strukturen haben sich in der Praxis besonders effektive Markierungen bewirken lassen.

Weiter bevorzugt im Sinne der Erfindung ist ein Verfahren, wobei die Verbindung V genau eine Dihydroxy- oder Trihydroxyphenylgruppe besitzt.

Dies hat den Vorteil, dass so über die absolute Konzentration der Strukturen V leicht auch die absolute Konzentration der zur Verfügung stehenden Catecholgruppen (einschließlich Trihydroxyphenylgruppen) kontrolliert werden kann.

Bevorzugt ist ein erfindungsgemäßes Verfahren, wobei die Verbindung V zusätzlich spezifische Bindungseigenschaften für die Zielstruktur oder Teile der Zielstruktur besitzt, bevorzugt in Form einer spezifischen Bindungsdomäne.

Bevorzugt sind im Rahmen dieses Textes "spezifische Bindungseigenschaften", dass die Verbindung V bei Vorhandensein der spezifischen Bindungseigenschaften mit einer um einen Faktor von wenigstens 10 erhöhten Wahrscheinlichkeit (bevorzugt unter Normalbedingungen) an einen bestimmten Bereich der Zielstruktur bindet, verglichen mit anderen Bereichen der Zielstruktur.

Weiter bevorzugt werden diese Eigenschaften in Form einer spezifischen Bindungsdomäne, d. h. ein Bereich als Bestandteil der Verbindung V, der aufgrund seiner räumlichen Struktur eine erhöhte Affinität zu einem bestimmten Bereich der Zielstruktur oder zu Strukturen auf der Oberfläche der Zielstruktur besitzt. Die Bindungsdomäne erhöht die Wahrscheinlichkeit der Anbindung an die Zielstruktur um mindestens den Faktor 10 verglichen mit einer gleichen Verbindung V ohne diese Bindungsdomäne. Ganz besonders bevorzugt handelt es sich bei der aufgrund der spezifischen Bindungseigenschaften bzw. der spezifischen Bindungsdomäne entstehenden Bindung um eine nicht-kovalente Bindung.

Eine solche spezifische Bindungsdomäne können z.B. sein: Rezeptorliganden (jeder Art), Peptidsequenzen, Kohlehydrate, Antikörper, RNA Sequenzen, DNA Sequenzen Antikörperfragmente, Enzymsubstrate, Enzyminhibitoren oder -effektoren sowie bindende Peptid- oder Proteinfragmente aus Phage Display Selektionen.

Bevorzugt ist entsprechend dem oben Gesagten ein erfindungsgemäßes Verfahren, wobei die spezifischen Bindungseigenschaften, insbesondere die spezifische Bindungsdomäne zur nicht-kovalenten Bindung, an die Zielstruktur eingerichtet sind. Hier lässt sich der Vorteil der (zusätzlich) kovalenten Bindung, die über das Chinon an geeignete Partner im Bereich der Zielstruktur erfolgen kann, besonders deutlich nutzen.

Bevorzugt ist ein erfindungsgemäßes Verfahren, wobei die Zielstruktur ausgewählt aus der Gruppe bestehend aus Zellen, Proteinen, Nukleinsäure, Virus, Hormon, Oligo-, Polysaccharid und Bio-Polymer.

Besonders bevorzugt ist ein erfindungsgemäßes Verfahren, bei dem die Verbindung V eine Signaldomäne umfasst.

Eine Signaldomäne im Sinne der vorliegenden Erfindung ist ein Bereich, der dazu dienen kann, mit einem geeigneten Signalermittlungsverfahren eine Markierung der Zielstruktur zu belegen, sie zu quantifizieren oder auszunutzen.

Bevorzugt ist in diesem Zusammenhang, dass die Signaldomäne eine Funktionalität umfasst, ausgewählt aus der Gruppe Fluorophor, Chromophor, Chemolumineszenzpartner, radioaktives Isotop, (para)magnetischer Bead, Nanopartikel, Enzym für Nachweisreaktion, aktivierte Sensoroberfläche und Affinitätsdomäne (zum Beispiel Antigensequenzen, Biotin, His-Tag).

Bevorzugte Verfahren, bei denen die Signalfunktion genutzt werden kann, sind Zellmarkierungen (FACS), Zelllabelling, Labelling mit Farbpigmenten, spezifischen Markierungen in vitro und in vivo, zum Beispiel mit Tumormarkern für bildgebende medizinische Verfahren

Bevorzugt ist auch ein erfindungsgemäßes Verfahren, wobei die Verbindung V eine zusätzliche Funktionsdomäne besitzt mit biologischer und/oder physikalischer Aktivität.

Auf diese Art ist es auch möglich, ein spezifisches Markieren mit einer Funktionsauslösung in der Zielstruktur zu koppeln. So ist es beispielsweise möglich, bestimmte Zelltypen, die sich durch spezifische Antigene an ihren Oberflächen auszeichnen, mit entsprechenden Funktionen zu belegen.

Dabei ist unter "biologischer Aktivität" der Funktionsdomäne zu verstehen, dass die Verbindung V einen Bereich umfasst, der Einfluss nimmt auf das Verhalten einer Zelle (z.B. über Integrine) oder auf einen Stoffwechselweg einer Zelle z. B. durch Blockieren, Aktivieren oder Beschleunigen.

"Physikalische Aktivität" bedeutet in diesem Zusammenhang, dass die zusätzliche Funktionsdomäne der Zielstruktur ein verändertes Verhalten hinsichtlich physikalischer Phänomene wie z. B. Fluoreszenz, Phosphoreszenz, Magnetismus oder Reaktion auf elektrische Reize vermittelt.

In diesem Sinne ist es bevorzugt, dass die Funktionsdomäne ausgewählt ist aus der Gruppe bestehend aus Apoptosesignalmolekül, Transkriptionsfaktor, DNA, Adjuvanz, Effektor für Signaltransduktion, Rezeptoragonist, -antagonist, kompetitiver Rezeptor-Effektor oder -Blocker sowie auch Kombinationen dieser Funktionalitäten.

So ist es möglich, zusätzlich zur spezifischen Markierung der Zielstruktur in der Zielstruktur oder an der Zielstruktur (weitere gewünschte) Effekte zu erzielen. So ist es z.B. denkbar, eine Zelle gezielt mit einem Apoptosesignal zu versehen, nachdem die spezifische Markierung stattgefunden hat.

Besonders bevorzugt ist ein erfindungsgemäßes Verfahren, wobei das Oxidieren der Dihydroxy- oder Trihydroxyphenylgruppe der Verbindung V zur Chinongruppe durch eine Lakkase erfolgt. Lakkasen arbeiten unter sehr milden (ggf. zellschonenden) Bedingungen, setzen den Aktivierungsschritt vom Di-/Tri-Phenol zum Chinon zuverlässig und schnell um und sind in vielen Umgebungen ausreichend stabil.

Ein besonderer Effekt des erfindungsgemäßen Verfahrens ist die Möglichkeit aus einem Zellgemisch spezieller Zelltypen zu gewinnen/isolieren, indem diese mit Hilfe einer für sie spezifischen Verbindung V markiert und nachfolgend selektiert werden.

Nachfolgend werden Beispiele für konkrete Anwendungen des erfindungsgemäßen Verfahrens aufgeführt:
So ist es möglich, Antikörper mit einem Reagenz aus einem Markierungsmolekül mit L-DOPA und einem erfindungsgemäß einzusetzenden Enzym zu markieren. Da es möglich ist, sehr spezifische Enzyme einzusetzen, die keine weitere Reaktion katalysieren und auch keine toxische Substanz verwendet wird, kann man, die markierten Antikörper ohne weitere Reinigung direkt einzusetzen. Besonders bevorzugte Anwendungsbereiche in diesem Zusammenhang sind IVD (In Vitro Diagnostica), Markierung von Zellen, schnelles Labelling mit Radionukleiden, *in vivo* Imaging.

Es ist beispielsweise auch möglich, das System für die Bindung von Peptiden einzusetzen: L-DOPA und andere Diphenole lassen sich wie Aminosäuren bei der Synthese der Peptide einbauen. So vorbereitete Peptide gehen nach der erfindungsgemäßen Aktivierung (Oxidierung der Dihydroxyphenylgruppe zum Chinon) schnell und irreversibel mit zum Beispiel Aminen in der nächsten Umgebung ihres (spezifischen) Bindungspartners, genauer ihrer spezifischen Bindungsstelle (also dem Bereich auf der Zielstruktur, der komplementär oder anders reaktiv mit der Bindungsdomäne ausgestaltet ist) eine kovalente Bindung ein. Dieses ist bevorzugt eine Struktur auf der Zielstruktur.

Besonders geeignet hat sich das erfindungsgemäße Verfahren für kovalente Markierung von Zellen (bzw. deren Zelloberflächen) gezeigt. So ist es möglich, Moleküle (bzw. Bindungsdomänen) einzusetzen, die zwar eine hohe Spezifizität für ein bestimmtes Zielmolekül besitzen, aber aufgrund einer hohen Dissoziationsrate beim Waschen einer solchen Präparation leicht wieder entfernt werden würden oder bei Verwendung einer zu hohen Konzentration der markierenden Verbindung ein zu hohes Hintergrundsignal des ungebundenen Moleküls verursachen.

Hierzu wird auch auf das unten stehende Beispiel verwiesen, bei dem die Kopplung eines DOPA- und Fluorescein markierten Peptids nach enzymatischer Aktivierung an Zelloberflächengebunden und anschließend FACS-Analyse oder Analyse unter dem Fluoreszenzmikroskop nachgewiesen werden konnte. Der Prozess der Aktivierung und der Markierung dauerte in den Experimenten nur wenige Minuten. Besonders vorteilhaft ist, dass die Markierung nicht wieder ausgetauscht wird, was sich durch die Stabilität der markierten Zellen auch in Gegenwart nicht-markierter Zellen beweisen lässt.

Ferner kann das vorliegende erfindungsgemäße Verfahren als schonende Alternative zur Markierung und anschließenden Affinitätsreinigung und Identifizierung von gebundenen Proteinen, zum Beispiel mit Massenspektroskopie dienen.

Verfahren zu denen das erfindungsgemäße Verfahren sich als eine Alternative erwiesen hat, sind das ProxiMol-Verfahren und ein Verfahren, das gemäß der WO 2004/064972 A9 offenbart ist, bei dem eine unphysiologische UV-Bestrahlung zum Koppeln erforderlich ist.

### Beispiele

### Beispiel 1

Markierung von Integrin αᵥβ₆ exprimierenden Zellen:

### Materialien

Zelltyp: Zelllinien HT1299 (Lungentumor), HUVEC, SVEC
Polypeptid zur Markierung: Ac-SPRGDLAVLGHK(FITC)GG-DOPA-G
(1-Buchstabencode für Peptide; FITC: Chromophor, Fluorescein, Anregungswellenlänge 490 nm, Emissionswellenlänge 520 nm)
Polypeptidlösung: 4 mg/ml Polypeptid in Wasser entsprechend 2 mM
20 mg/ml Lakkase aus Trameters versicolor, 20 U/mg, ASA Spezialenzyme GmbH, Wolfenbüttel
NaAc-Lösung, 3 M, pH 5,6
Alle FACS Messungen wurden an einem Cytomics FC500 (Beckman Coulter) durchgeführt.

### Vorbereitung der Testansätze:

Die Zellen wurden jeweils zweimal mit PBS gewaschen und nachfolgend zu ca. 10⁵ Zellen/ml in PBS aufgenommen. Von dieser Lösung wurden jeweils 950 µl in ein 1-ml-FACS-Röhrchen gegeben. Dazu wurden nach der unten gegebenen Tabelle 1 markierte Polypeptide mit und ohne Vorbehandlung durch Lakkase gegeben.

Hierzu wurden in einem typischen Ansatz für 5 µM Polypeptid 40 µl NaAc-Lösung, 105 µl Wasser, 15 µl Peptidlösung und 120 µl Lakkase gegeben. Proben ohne Lakkase enthielten das entsprechende zusätzliche Volumen Wasser, Proben mit anderen Polypeptidkonzentrationen entsprechend mehr Polypeptidlösung (vgl. Beispiel 2). Nach 1 min Inkubationszeit wurden je 50 µl zu den Zellsuspensionen gegeben oder in den FACS Röhrchen vorgelegt.

Nach einer Inkubationszeit von mindestens 15 Minuten wurden die Zellen dreimal mit 2 ml PBS im Röhrchen gewaschen und anschließend am Zellsorter die Fluoreszenz der Zellen gemessen. Gemessen wurde die Gesamtfluoreszenz der Zellen. Eine Auswahl bestimmter Zelltypen veränderte das Gesamtergebnis nicht wesentlich.

### Versuchsansätze zur Hintergrundbindung:

**Tabelle 1**

| **Experiment** | **Zelltyp** | **Beschreibung** |
|---|---|---|
| 99-K0 | HT1299 | Nur PBS (Kontrolle ohne Laccase) |
| 99-K5 | HT1299 | PBS mit 5 µM Polypeptid (Kontrolle ohne Laccase) |
| 99-5 | HT1299 | 5 µM Polypeptid mit Laccase |
| SV-K0 | SVEC | Nur PBS (Kontrolle ohne Laccase) |
| SV-K5 | SVEC | PBS mit 5 µM Polypeptid (Kontrolle ohne Laccase) |
| SV-5 | SVEC | 5 µM Polypeptid mit Laccase |

### Ergebnis:

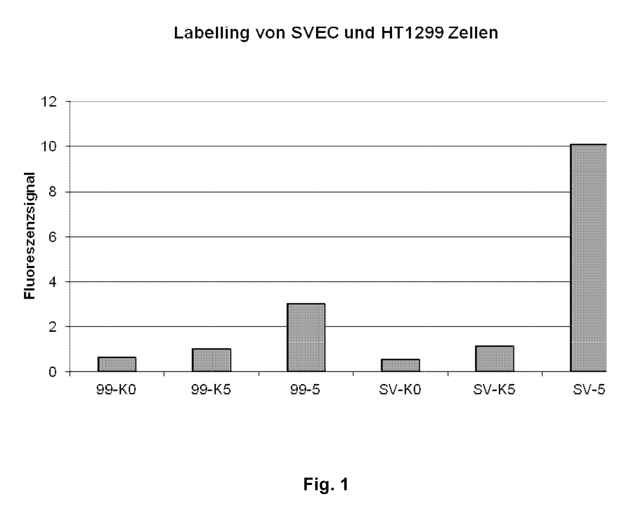

Die Ergebnisse sind in Fig. 1 dargestellt und zeigen:
Ohne Lakkasezugabe lässt sich das Polypeptid wieder von den Zellen abwaschen.

### Beispiel 2

### Versuchsansätze zur Abhängigkeit der Bindung von der Polypeptidkonzentration:

Entsprechend der obigen Versuchsdurchführung (Beispiel 1) wurden verschiedene Zelllinien die alle das αᵥβ₆ Integrin exprimieren mit verschiedenen Polypeptidkonzentrationen markiert.

### Ergebnis:

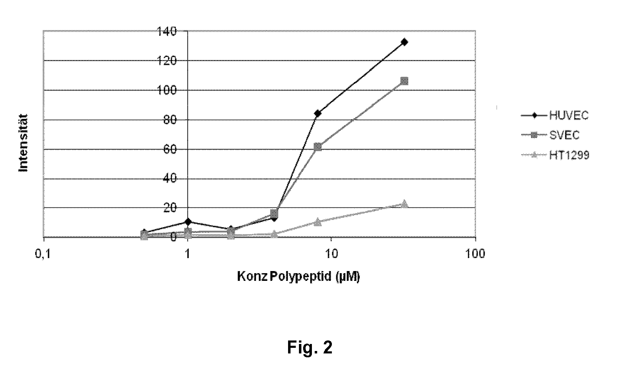

Die Ergebnisse sind in Fig. 2 abgebildet:
Die Polypeptide binden konzentrationsabhängig, das heißt sie zeigen eine echte Bindungskurve obwohl sie irreversibel (kovalent) gebunden wurden. Das bedeutet zum einen, dass die oxidierte biphenolische Gruppe rasch reagiert, wenn das Polypeptid (spezifisch) gebunden ist, und zum anderen dass diese Gruppe nicht so schnell reagiert, dass beliebige Aminogruppen markiert werden.

### Gesamtbewertung

Es ist praktisch möglich, eine Markierung *in situ* vorzunehmen, bei der das oxidierende Enzym (hier Lakkase), die Verbindung V (hier das Polypeptid) sowie zu markierende Zielstruktur (hier auf verschiedenen Zelltypen) in ein Reaktionsgefäß gegeben werden. Es hat sich aber herausgestellt, dass eine kurze Präinkubation, zum Beispiel eine Minute, der Lakkase mit dem Polypeptid (zur Aktivierung) einen vorteilhaften Effekt auf die Markierungseffizienz besitzt. Dies dürfte auch auf parallele Ansätze im Sinne der Erfindung übertragbar sein. Die hier gewählten Bedingungen mit einem leicht sauren pH wurden bevorzugt gewählt um nahe am Optimum des Enzyms zu arbeiten. Aus praktischen Messungen mit Substraten ist aber bekannt, dass wesentlich niedrigere Enzymkonzentrationen und auch ein pH im neutralen Bereich ausreichend sind.

### Beispiel 3

Kopplung von Strukturen mit DOPA-Rest an ein Protein

Die folgenden Lösungen wurden in 10 mM Phosphatpuffer pH 7,0 zubereitet:
a) Hühnereiweiß Lysozym 1 mg/ml, ensprechend 70 µM (Lösung 1)
b) Verbindung A (Biotin-DOPA) 1 mg in 3mL, entsprechend 700 µM (Lösung 2) und eine 70 µM Verdünnung (Lösung 3)
c) Verbindung B (Biotin-RGD-DOPA) 1,2 mg in 2 ml, entsprechend 700 µM (Lösung 4) und eine 70 µM Verdünnung (Lösung 5)
d) Laccase (Trametes vers., ASA Spezialenzyme GmbH) 1 mg/ml entsprechend 20 u/ml(Lösung 6)
   Verbindung A 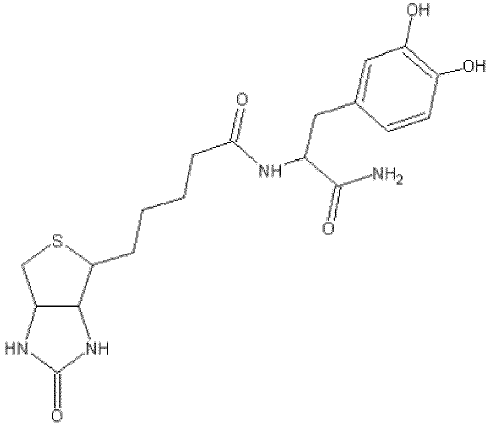
      Summenformel = C₁₉H₂₆N₄O₅S
      Molgewicht = 422,4997 g/mol
   Verbindung B 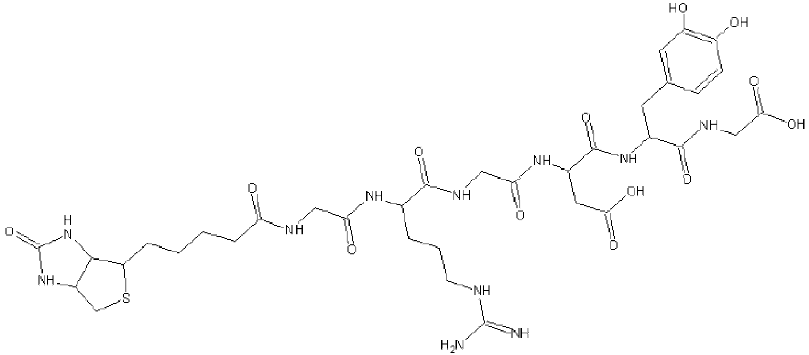
      Summenformel = C₃₅H₅₁N₁₁O₁₃S
      Molgewicht = 865,9118 g/mol

Die Reaktion wurde durch Mischen von je 500 µl Lysozym Lösung (Lösung 1) mit je 500 µl von Lösung 2 bis Lösung 5 (also jeweils equimolar und zehnfacher Überschuß) und Zugabe und Untermischen von je 10 µl / 0,2 u Laccase (Lösung 6) gestartet. Als Kontrolle wurde Lysozym mit 500 µl 10mM Phosphatpuffer pH 7,0 und Laccase (Lösung 6) angesetzt. Die Proben wurden über Nacht bei Raumtemperatur inkubiert und am nächsten Tag wurde ein Aliqot über eine mit deionisiertem Wasser equilibrierte Entsalzungssäule (Micro Bio-Spin P6, BioRad) von niedermolekularen Bestandteilen und Salzen befreit.

Das so vorbereitete Produkt der Reaktion wurde in einem AB Sciex TOF/TOFTM 5800 System analysiert.

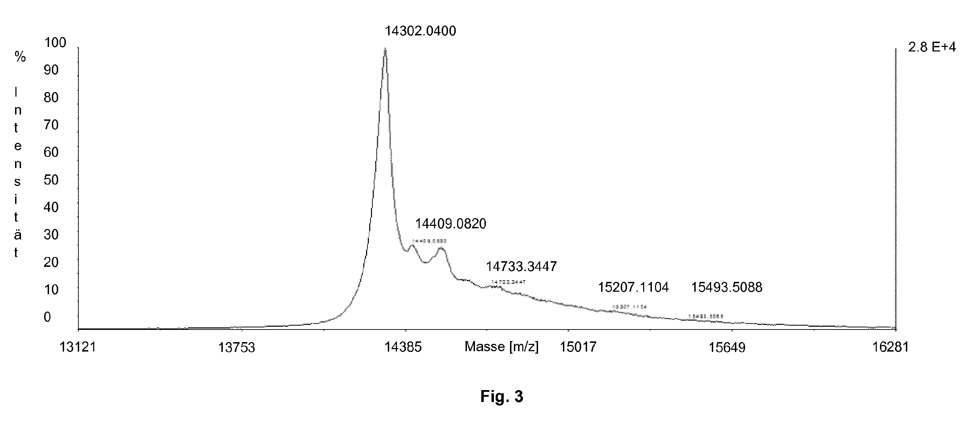

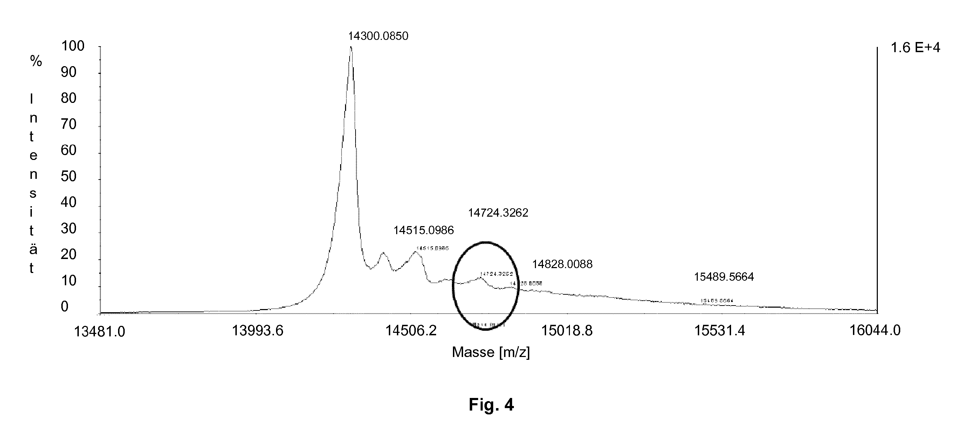

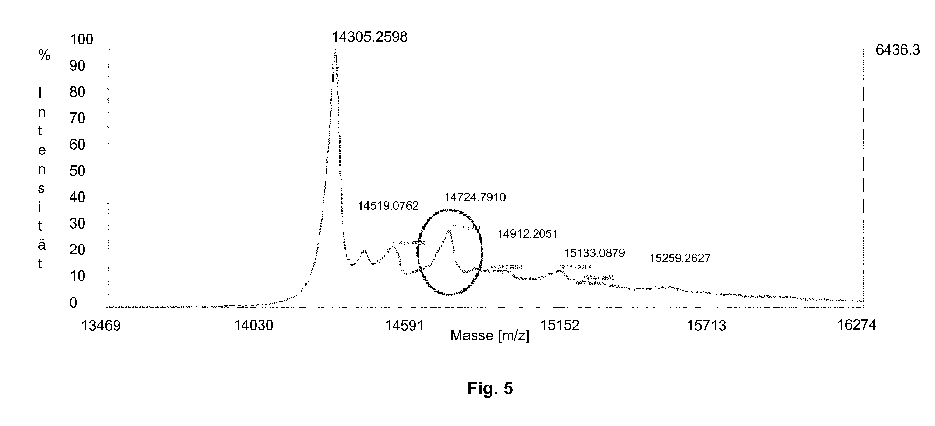

Figuren 3 bis 5 zeigen: MS-Analyse der Reaktion von Verbindung A mit Lysozym; Fig. 3 Kontrollprotein, Fig. 4; equimolar und Fig. 5; zehnfacher Überschuss. Die beobachteten Massen für das Lysozym bzw Addukt 14300 Da zu 14724,3 Da (b) bzw 14302 Da zu 14724,8 Da (c) decken sich mit dem erwarteten Zuwachs von 418,5 Da.

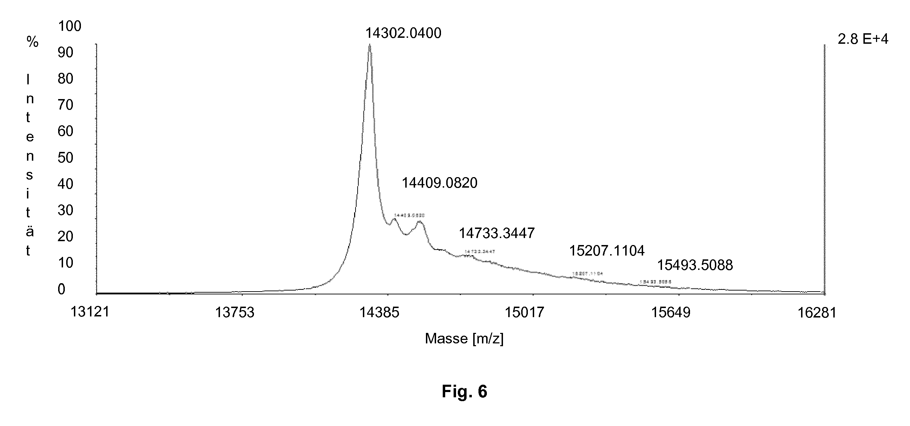

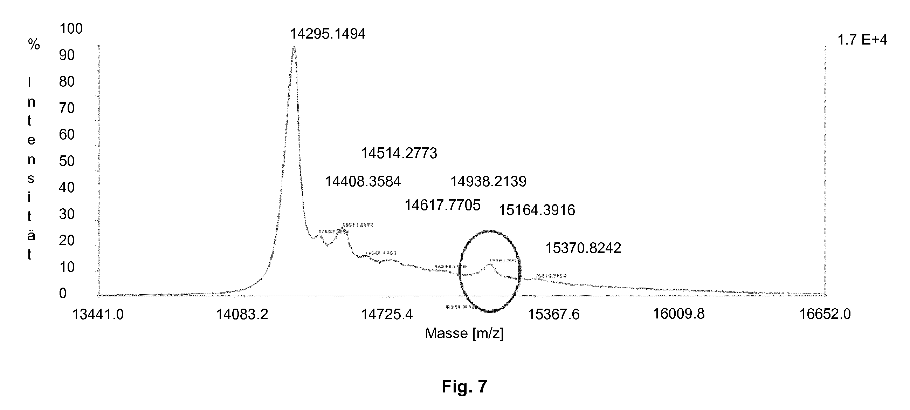

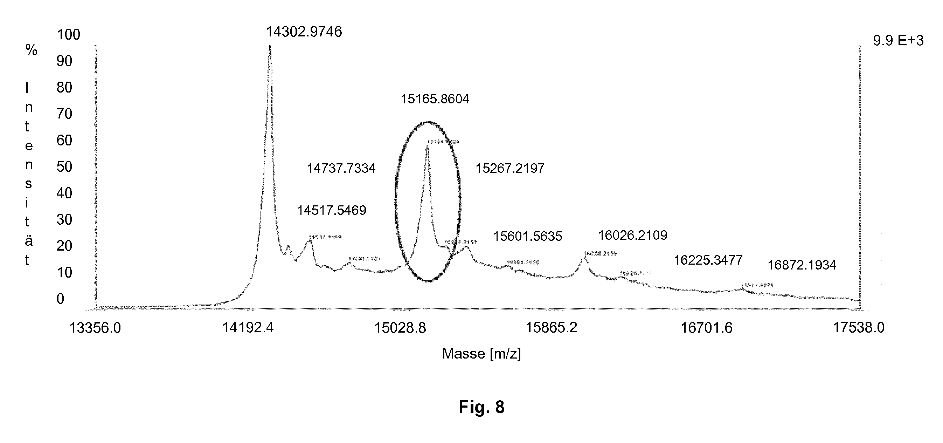

Figuren 6 bis 8 zeigen: MS-Analyse der Reaktion von Verbindung B mit Lysozym; Fig. 6 Kontrollprotein, Fig. 7 equimolar und Fig. 8 zehnfacher Überschuss. Die beobachteten Massen für das Lysozym bzw Addukt 14295 Da zu 15164,4 Da (b) bzw 14302 Da zu 15167,9 Da (c) decken sich mit dem erwarteten Zuwachs von 861,9 Da.

Die erwarteten Reaktionsprodukte wurden in allen Fällen gefunden und die Massenspektren (Figuren 5 und 8) zeigen, dass auch bei Überschuss der DOPA-Verbindungen A und B ein vollständiger Umsatz nicht erreicht wurde. Lysozym wird auch bei zehnfachem Überschuss der DOPA-Verbindung kaum doppelt markiert.

Schlussfolgerungen:
1. Die Reaktion findet wie erwartet statt
2. Die Daten lassen die begründete Annahme zu, dass die Laccase-aktivierte Verbindung auch im Überschuss nicht übermäßig schnell und vollständig mit dem Protein reagiert. Das erklärt auch die Möglichkeit einer verbesserten Reaktion bei spezifisch bindenden Peptiden am Beispiel des Integrin-bindenden Peptids in Beispiel 1 und 2.

## Patentansprüche

1. Verfahren zum Markieren einer Zielstruktur, umfassend die Schritte:
a) Bereitstellen einer Verbindung V, die wenigstens eine Dihydroxy- oder Trihydroxyphenylgruppe umfasst,
b) Bereitstellen eines Mittels zum Umwandeln der Dihydroxy- oder Trihydroxyphenylgruppe zu einer Chinongruppe,
c) Bereitstellen einer biologischen Zielstruktur,
d) Oxidieren der Dihydroxy- oder Trihydroxyphenylgruppe der Verbindung V zur Chinongruppe und
e) Kontaktieren der Verbindung V mit der biologischen Zielstruktur zum Markieren derselben, so dass eine kovalente Verbindung gebildet werden kann,
**dadurch gekennzeichnet,**
**dass** im Schritt a) die Verbindung V eine spezifische Erkennungssequenz für die biologische Zielstruktur umfasst,
**dass** im Schritt b) das Mittel ein Enzym, vorzugsweise eine Oxidoreduktase, ist, und
**dass** im Schritt e) die Verbindung V in einer Konzentration eingesetzt wird, so dass die maximale Konzentration an Dihydroxy-, Trihydroxyphenylgruppen und Chinongruppen, die durch die Verbindung V eingebracht wurden, ≤ 500µM, bevorzugt ≤ 300µM, weiter bevorzugt ≤ 100µM ist.

2. Verfahren nach Anspruch 1, wobei die wenigstens eine Dihydroxy- oder Trihydroxyphenylgruppe Bestandteil von DOPA, Dopamin, Kaffeesäure oder einer entsprechenden gebundenen Gruppe aus einer dieser Verbindungen ist.

3. Verfahren nach Anspruch 1 oder 2, wobei die Verbindung V genau eine Dihydroxy- oder Trihydroxyphenylgruppe besitzt.

4. Verfahren nach einem der vorangehenden Ansprüche, wobei die Verbindung V zusätzlich spezifische Bindungseigenschaften für die Zielstruktur oder Teile der Zielstruktur besitzt, bevorzugt in Form einer spezifischen Bindungsdomäne.

5. Verfahren nach Anspruch 4, wobei die spezifische Bindungseigenschaften zur nicht-kovalenten Bindung an die Zielstruktur eingerichtet ist.

6. Verfahren nach einem der vorangehenden Ansprüche, wobei die Zielstruktur ausgewählt ist aus der Gruppe bestehend aus Zelle, Protein, Nucleinsäure, Virus, Hormon, Oligo-, Polysaccharid und Bio-Polymer.

7. Verfahren nach einem der vorangehenden Ansprüche, wobei die Verbindung V eine zusätzliche signalgebende Struktur umfasst.

8. Verfahren nach Anspruch 7, wobei die signalgebende Struktur ausgewählt ist aus der Gruppe Fluorophor, Chromophor, Chemolumineszenzpartner, radioaktives Iso-top, magnetischer Bead, aktivierte Sensoroberfläche, Enzym für Nachweisreaktion, aktivierte Sensoroberfläche und Affinitätsdomäne.

9. Verfahren nach einem der vorangehenden Ansprüche, wobei die Verbindung V eine zusätzliche Funktionsdomäne besitzt mit biologischer und/oder physikalischer Aktivität.

10. Verfahren nach Anspruch 9, wobei die Funktionsdomäne ausgewählt ist aus der Gruppe bestehend aus Apoptosesignalmolekül, Transkriptionsfaktor, DNA, Adjuvanz, Effektor für Signaltransduktion, Rezeptoragonist, -antagonist, kompetitiver Rezeptor-Effektor oder-Blocker sowie auch Kombinationen dieser Funktionalitäten.

11. Verfahren nach einem der vorangehenden Ansprüche, wobei das Oxidieren der Dihydroxy- oder Trihydroxyphenylgruppe der Verbindung V zur Chinongruppe durch eine Lakkase erfolgt.

12. Verwendung einer Verbindung V wie in einem der vorangehenden Ansprüche definiert zum Markieren einer biologischen Zielstruktur wobei ein enzymatisches Oxidieren der Dihydroxy- oder Trihydroxyphenylgruppe der Verbindung V zur Chinongruppe erfolgt.

## Claims

1. A Method for marking of a target structure comprising the steps of:
a) providing a compound V comprising at least one dihydroxy or trihydroxyphenyl group,
b) providing an agent for converting the dihydroxy or trihydroxyphenyl group into a quinone group,
c) providing a biological target structure,
d) oxidizing the dihydroxy or trihydroxyphenyl group of the compound V into the quinone group and
e) contacting the compound V with the biological target structure for marking the same, so that a covalent bond can be formed,
**characterized in**
**that** in step a) the compound V comprises a specific recognition sequence for the biological target structure
**that** in step b) the agent is an enzyme, preferably an oxido reductase, and that in step e) the compound V is applied in a concentration, so that the maximum concentration of dihydroxy, trihydroxyphenyl groups and quinone groups, which were introduced by the compound V, is ≤ 500µM, preferably ≤ 300µM, more preferably ≤ 100µM.

2. The method according to claim 1, wherein the at least one dihydroxy or trihydroxyphenyl group is component of DOPA, dopamine, caffeic acid or a corresponding bonded group of one of these compounds.

3. The method according to claim 1 or 2, wherein the compound V has exactly one dihydroxy or trihydroxyphenyl group.

4. The method according to any of the preceding claims, wherein the compound V in addition has specific binding properties for the target structure or parts of the target structure, preferably in form of a specific binding domain.

5. The method according to claim 4, wherein the specific binding property is arranged for the non-covalent binding to the target structure.

6. The method according to any of the preceding claims, wherein the target structure is selected from the group consisting of cell, protein, nulcleic acid, virus, hormone, oligo-, polysaccharide and bio-polymer.

7. The method according to any of the preceding claims, wherein the compound V comprises an additional signal-generating structure.

8. The method according to claim 7, wherein the signal-generating structure is selected from the group fluorophore, chromophore, chemiluminescence partner, radioactive isotop, magnetic bead, activated sensor surface, enzyme for detection reaction, activated sensor surface and affinity domain.

9. The method according to any of the preceding claims, wherein the compound V has an additional functional domain with biological and/or physical activity.

10. The method of claim 9, wherein the functional domain is selected from the group consisting of apoptosis signaling molecule, transcription factor, DNA, adjuvant, effector for signal transduction, receptor-agonist, -antagonist, competitive receptor-effector or -blocker as well as combinations of these functionalities.

11. The method according to any of the preceding claims, wherein the oxidizing of the dihydroxy or trihydroxyphenyl group of the compound V into the quinone group is effected by a laccase.

12. Use of a compound V as defined in any of the preceding claims for marking a biological target structure, wherein an enzymatic oxidizing of the dihydroxy or trihydroxyphenyl group of the compound V into the quinone group is effected.

## Revendications

1. Procédé pour marquer une structure cible, comprenant les étapes suivantes consistant à :
a) fournir un composé C, qui comprend au moins un groupe dihydroxy ou trihydroxyphényle,
b) fournir un moyen pour transformer le groupe dihydroxy ou trihydroxyphényle en un groupe quinone,
c) fournir une structure cible biologique,
d) oxyder le groupe dihydroxy ou trihydroxyphényle du composé C en le groupe quinone et
e) mettre en contact le composé C avec la structure cible biologique afin de marquer celle-ci de manière à pouvoir former une liaison covalente, **caractérisé en ce**
**que** le composé C comprend, à l'étape a), une séquence de reconnaissance spécifique pour la structure cible biologique,
**que** le moyen est, à l'étape b), une enzyme, de préférence une oxydoréductase, et
**que** le composé C est utilisé, à l'étape e), en une concentration de telle sorte que la concentration maximale en groupes dihydroxy et trihydroxyphényle et en groupes quinone, qui sont introduits par le composé C, est ≤ 500 µm, de manière préférée ≤ 300 µM, de manière davantage préférée de 100 µM.

2. Procédé selon la revendication 1, dans lequel l'au moins un groupe dihydroxy ou trihydroxyphényle est un constituant de la DOPA, de la dopamine, de l'acide caféique ou d'un groupe lié correspondant composé de l'un desdits composés.

3. Procédé selon la revendication 1 ou 2, dans lequel le composé C possède précisément un groupe dihydroxy ou trihydroxyphényle.

4. Procédé selon l'une quelconque des revendications précédentes, dans lequel le composé C possède en supplément des propriétés de liaison spécifiques pour la structure cible ou des parties de la structure cible, de manière préférée sous la forme d'un domaine de liaison spécifique.

5. Procédé selon la revendication 4, dans lequel les propriétés de liaison spécifiques sont mises au point aux fins de la liaison non-covalente à la structure cible.

6. Procédé selon l'une quelconque des revendications précédentes, dans lequel la structure cible est choisie parmi le groupe constitué de cellule, de protéine, d'acide nucléique, de virus, d'hormone, d'oligosaccharide, de polysaccharide et de biopolymère.

7. Procédé selon l'une quelconque des revendications précédentes, dans lequel le composé C comprend une structure supplémentaire émettant des signaux.

8. Procédé selon la revendication 7, dans lequel la structure émettant les signaux est choisie parmi le groupe comprenant du fluorophore, du chromophore, un partenaire de chimioluminescence, un isotope radioactif, une bille magnétique, une surface activée de capteur, une enzyme de réaction de détection, une surface activée de capteur et un domaine d'affinité.

9. Procédé selon l'une quelconque des revendications précédentes, dans lequel le composé C possède un domaine fonctionnel supplémentaire comprenant une activité biologique et/ou physique.

10. Procédé selon la revendication 9, dans lequel le domaine fonctionnel est choisi parmi le groupe constitué de la molécule de signalisation d'apoptose, du facteur de transcription, de l'ADN, de l'adjuvant, de l'effecteur de transduction de signal, de l'agoniste récepteur, de l'antagoniste récepteur, de l'effecteur ou bloqueur récepteur compétitif ainsi que de combinaisons desdites fonctionnalités également.

11. Procédé selon l'une quelconque des revendications précédentes, dans lequel l'oxydation du groupe dihydroxy ou trihydroxyphényle du composé C en le groupe quinone est effectuée par une lactase.

12. Utilisation d'un composé C tel que défini dans l'une quelconque des revendications précédentes afin de marquer une structure cible biologique, dans laquelle une oxydation enzymatique du groupe dihydroxy ou trihydroxyphényle du composé C en le groupe quinone est effectuée.
